(19) Europäisches Patentamt
European Patent Office
Office européen des brevets

(11) **EP 0 913 399 A1**

(12) **EUROPEAN PATENT APPLICATION**

(43) Date of publication:
**06.05.1999 Bulletin 1999/18**

(51) Int. Cl.[6]: **C07F 1/10**, A01N 59/16, C07C 323/62, A61K 31/28

(21) Application number: **97119160.6**

(22) Date of filing: **03.11.1997**

(84) Designated Contracting States:
**AT BE CH DE DK ES FI FR GB GR IE IT LI LU MC NL PT SE**
Designated Extension States:
**AL LT LV RO SI**

(71) Applicant:
**Korban Medical S.a.s. di Longhetti Gianna & C.**
**10132 Turin (IT)**

(72) Inventor: **Silvola, Alessandro**
**10132 Turin (IT)**

(74) Representative:
**Robba, Pierpaolo et al**
**Interpatent,**
**Via Caboto 35**
**10129 Torino (IT)**

(54) **Silver -2-mercapto sodium benzoate pharmaceutical compound and process for obtaining the same**

(57) There are disclosed a silver-2-mercapto sodium benzoate pharmaceutical compound having the formula (I)

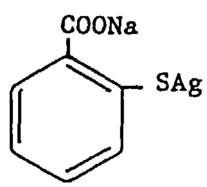

(I)

and a process for obtaining such compound.

EP 0 913 399 A1

## Description

The present invention refers to a silver mercaptide pharmaceutical compound and to a process for obtaining such compound.

More precisely the invention relates to a silver-2-mercapto sodium benzoate having the following formula (I)

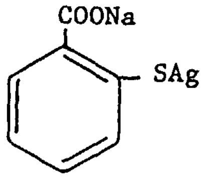

(I)

the process for its preparation and its use as a broad-spectrum topic antibacterial.

In the chemical and pharmaceutical literature the germicidal properties of many heavy metals, particularly silver, are well known.

Such properties are ascribable to the deep irreversible structural modifications (precipitation, coagulation, alterations) caused by the metal to the proteinic material forming the cytoplasmic membrane of the bacterial cell.

However the compounds derived from silver salts that are commonly employed in pharmacology frequentely have the drawback of staining the skin and the undergarments (e.g. the silver nitrate) and can be caustic and/or irritating to the skin, often causing photosensitization effects.

In dermatology there are further employed other silver derivatives associated with sulphonamide derivatives that in general do not present the drawback of staining the skin and the undergarments.

However the presence of sulphonamide is unadvisable for example during pregnancy, for babies and in case of extensive burns that can cause a systemic absorption with possible kidney damages.

It has now been found, and it is the object of the present invention the compound silver-2-mercapto sodium benzoate that can be obtained through complexation of an alkaline salt with a thioacid (sodium salt of the 2-mercaptobenzoic acid) and a salt that is soluble in silver in a aqueous-acetonic-alcoholic phase.

Such a compound has remarkable antibacterial properties (better than those found in the common silver salts), is without caustic and/or irritating effects, without photosensitization effects and colourless.

The preparation of silver-2-mercapto sodium benzoate is illustrated by the following reaction:

$$HS\text{-}C_6\text{-}H_4\text{-}COONa + AgNO_3 \rightarrow AgS\text{-}C_6\text{-}H_4\text{-}COONa + HNO_3$$

and consists of a double exchange mercaptide complexation (1:1 mol) between silver-2-mercapto sodium benzoate and silver in a aqueous-acetonic-alcoholic phase.

The so obtained product has a yield higher than 95%.

The process according to the invention is advantageous in view of its mild reaction conditions and can easily be transferred onto an industrial scale.

The following example further illustrates the invention.

EXAMPLE

157.3 g of 2-mercaptobenzoic acid 98% Fluka (1 gmol x factor 100%) were treated in a 2 lt flask immersed in a bath cooled with crushed ice and salt with 500 ml of alcoholic NaOH 2N; then 200 ml of acetone were added to the solution to obtain a mixture with a pH 6÷7: solution (A).

In another flask 171.6 g of silver nitrate 99% Fluka (1 gmol x factor 100%) were dissolved without heating in 170 ml of $H_2O$: solution (B).

A mechanical blade stirrer was fitted to the flask containing solution (A) and then solution (B) was slowly added in the form of a thin trickle.

In this phase a citrine yellow precipitate of silver-2-mercapto sodium benzoate monomer (Na/Ag(TSA)/.n$H_2O$) began to form at once.

At the end of the reaction the precipitate and the mother liquor had a characteristic citrine yellow colour.

An excess of acetone was added and the mixture was kept under N or $CO_2$ atmosphere in a closed dark vessel.

The mixture was vacuum filtered on Büchner with paper of the Whatman type interposed; it was washed with

further acetone (diethylether); the solvent was evaporated in a drier in which cold air circulated; then was kept in a vacuum drier (700/750 mm Hg) for 12 hours.

Finally the obtained product was then kept in a closed dark glass jar under N or $CO_2$ atmosphere.

The yield of usable product was 274 g of anhydrous product, corresponding to 96.5%, and 295.7 g of hydrated product.

Chemical development of the obtained product

- Chemical structure: monomeric product

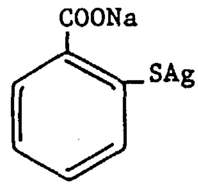

(anhydrous or hydrated)

molecular formula: AgS-$C_6$ -$H_4$ -COONa ($H_2O$)
relative molecular mass (Mr): 283.052 (anhydrous)

- chemical denomination: 2-mercaptide-benzene-1 -sodium carboxylate, silver-sodium thiosalicylate.

| - calculated composition | anhydrous | found |
|---|---|---|
| 2-mercaptobenzoic acid | 53.76% | 52.08% |
| sodium | 8.112% | - - |
| silver ($Ag^+$) | 38.109% | 37.92% |
| water | - - | 8.1% |
| (mercapto sodium benzoate) | 62.24% | 62.10% |

- Chemical-physical properties:

Physical form and organoleptic properties: cristalline powder, citrine yellow color, fluorescent; odourless, does not cause photosensitization, irritating or caustic effects to the skin.

- Polymorphism: monomorphic (monoclinic crystal)
- Polymerization: monomer (also the orange colored dimer exists, depending on the sodium used in the preparation). Solubility: soluble in water (cold), moderately soluble in DMSO, insoluble in ether, acetone, absolute ethanol.
- Melting point: (anhydrous, within a closed tube) 302° with decomposition.
- pH (1% solution in $H_2O$): 8.1

Identification

= Test for $Ag^+$ (ionic): chromatographic TLC ascending test on cellulose MN 300, against standard silver (nitrate) 0.1 N Aldrich cod. 31,943-0, ref.: K. Randerath: "Thin Layer Chromatography", page 255: positive.

= $Na^+$ Test: flame photometric test, against standard: positive.

"NaHTSA binder" test: test Ann. Franç XIX, 81, 1961 for the radical HS- : positive.

= IR spectrum test on KBr (against standard): corresponding

Assay

= Test for $Ag^+$ (ionic): test according to Volhard: g 37.92% d.s. (theoretical g 38.109% d.s.)

= Test for $Ag^+$ (ionic): plasma emission spectrophotometric test with reading of the results at 328.068 nm, against standard 20-300 mcg $Ag^+$/l sol.: g 37.84% (error ± 3.468%).

Evaluation of the antibacterial activity "in vitro"

The use of the salt of a thiometal (2-mercapto benzoic acid) allows to obtain a broad-spectrum compound based on $Ag^+$, the antibacterial activity of which has been tested in vitro (European pharmacopoeia test) with successful results, particularly on Gram- negative, Gram-positive, yeasts, moulds: Escherichia Coli, Klebsiella pneumoniae, Salmonella anatum, Proteus vulgaris, Proteus mirabilis, Pseudomonas aeruginosa; Staphylococcus aureus, Bacillus subtilis, Bacillus antracis, Staphylococcus piogenes, Streptococcus faecalis; Saccaromices cerevisiae, Candida albicans.

Experimentally the mercapto sodium benzoate anion does not develop an antibacterical action, whereas a comparison between the activity of equivalent ionic doses of $Ag^+$ from thiometal and from nitrate detects an activity in MIC(mcg/ml) variable depending on the tested organism in favour of the thiometal (from -1.6 to -50).

The above disclosed production technology can be adapted to other similar thiometals such as Ag-n sodium acetil cisteinate and others.

In view of the effective topic antibacterial activity and the absence of caustic and/or irritating effects, of photosensitization effects, the absence of systemic effects, it falls within the scope of the present invention the use of silver-2-mercapto sodium benzoate as agent for both preventing and treating dermatological infections caused by silver sensitive germs on mucosae, skin intact (PMC) and injured because of traumas, burns, skin sores, eg. bedsores, ulcerations, etc.

The compound of the invention can be carried both in liquid or solid phase (solutions, powders, sprays or suspensions, stiff gel compresses, gauzes creams) with auxiliary carriers that are dermocompatible, either alone or in association for better exploiting the spectrum antisepticity or with skin protective products that are specific for tissue repair (eg derivatives of jaluronic and/or aginic acids).

## Claims

1. A pharmaceutical compound characterized by being constituted by silver-2-mercapto sodium benzoate having the following formula (I)

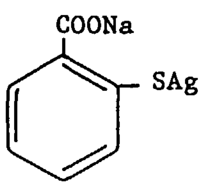

(I)

2. A pharmaceutical compound as claimed in claim 1, characterized in that it further comprises derivatives of jaluronic acid and/or alginic acid.

3. A process for preparing silver-2-mercapto sodium benzoate having the formula (I)

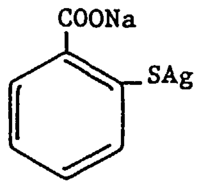

(I)

characterized in that it comprises a double exchange mercaptidic complexation (1:1 mol) between an alkaline salt of a thioacid and a silver salt soluble in aqueous-acetonic-alcoholic phase.

**4.** A process as claimed in claim 3, characterized in that it comprises the steps of:

a) preparing a sodium salt of the 2-mercapto benzoic acid in aqueous-acetonic-alcoholic phase without heating, solution (A), by adding NaOH in alcoholic phase to the 2-mercapto benzoic acid;
b) solubilizing (without heating) $AgNO_3$ in aqueous phase, solution (B);
c) adding solution (B) to solution (A) while stirring the latter;
d) precipitating a silver-2-mercapto sodium benzoate monomer (Na/Ag(TSA)/.n$H_2O$);
e) filtering the precipitate obtained in the preceding step, washing the filtrate and drying the washed filtrate.

European Patent Office

# EUROPEAN SEARCH REPORT

Application Number
EP 97 11 9160

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | K. NOMIYA ET AL.: "SYNTHESIS AND CHARACTERIZATION OF POLYMERIC, ANIONIC THIOSALICYLATO-AG(I) COMPLEXES WITH ANTIMICROBIAL ACTIVITIES" JOURNAL OF INORGANIC BIOCHEMISTRY, vol. 58, no. 4, 1995, pages 255-267, XP002062429 see the whole document, in particular page 259, last paragraph --- | 1-4 | C07F1/10 A01N59/16 C07C323/62 A61K31/28 |
| X | EP 0 727 427 A (MEIJI MILK PROD CO LTD ;TOYO INK MFG CO (JP)) 21 August 1996 * abstract; claims; example 2; tables 2,7 * --- | 1-4 | |
| X | CHEMICAL ABSTRACTS, vol. 123, no. 12, 18 September 1995 Columbus, Ohio, US; abstract no. 159461, O. MUNEHIRO ET AL.: "PREPARATION OF THIOSALICYLIC ACID SILVER COMPLEXES AS ANTIBACTERIAL AND ANTIVIRAL AGENTS" XP002062432 * abstract * & DATABASE WPI Week 9503 Derwent Publications Ltd., London, GB; AN 95-019264 & JP 06 306 082 (MEIJI MILK PROD CO LTD) , 1 November 1994 * abstract * --- | 1-4 | TECHNICAL FIELDS SEARCHED (Int.Cl.6) C07F A01N C07C A61K |
| A | EP 0 037 318 A (FABRE SA PIERRE) 7 October 1981 * abstract * * page 2, line 21 - page 3, line 2; claims; example 5 * --- -/-- | 1-4 | |

The present search report has been drawn up for all claims

| Place of search | Date of completion of the search | Examiner |
|---|---|---|
| THE HAGUE | 17 April 1998 | Hoff, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

European Patent Office

# EUROPEAN SEARCH REPORT

Application Number
EP 97 11 9160

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | A. BULT ET AL.: "REMARKS ON THE STRUCTURE-ACTIVITY OF SOME ANTIBACTERIAL SILVER NON-SULFANILAMIDE COMPOUNDS" PHARMACEUTISCH WEEKBLAD SCIENTIFIC EDITION, vol. 3, no. 2, 1981, pages 79-81, XP002062430 * the whole document * --- | 1-4 | |
| A | M. AL-DAHER: "1H NMR STUDY OF SOME THIOSALICYLIC ACID COMPLEXES" SPECTROSCOPY LETTERS, vol. 23, no. 6, 1990, pages 791-799, XP002062431 * the whole document * --- | 1-4 | |
| A | GB 465 291 A (G.W. JOHNSON) * example 2 * --- | 1-4 | |
| A | GB 398 020 A (SCHERING) * example 9 * ----- | 1 | TECHNICAL FIELDS SEARCHED (Int.Cl.6) |

The present search report has been drawn up for all claims

| Place of search | Date of completion of the search | Examiner |
|---|---|---|
| THE HAGUE | 17 April 1998 | Hoff, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)